# EUROPEAN PATENT SPECIFICATION

(11) **EP 4 475 919 B1**
(45) Date of publication and mention of the grant of the patent: **15.04.2026**
(21) Application number: 23706070.2
(22) Date of filing: 08.02.2023
(51) Int. Cl.: A61M 11/02, A61M 15/00

(54) **INHALER**
INHALATOR
INHALATEUR

(30) Priority: 08.02.2022 GB 202201567; 19.08.2022 GB 202212133
(43) Date of publication of application: 18.12.2024
(73) Proprietor: 1nhaler Ltd, Edinburgh Lothian EH6 4JR (GB)
(72) Inventor: BAUMHAKL, Patrick, 71560 Sulzbach (DE); WOLBERS, Kai, 71397 Leutenbach (DE); SMITH, Donald, Edinburgh Lothian EH6 4JR (GB)
(74) Representative: HGF
(86) International application number: PCT/GB2023/050277
(87) International publication number: WO 2023/152482

(56) References cited:
- WO-A1-2019/008336
- WO-A1-2021/191606
- US-A- 6 098 619
- US-A1- 2020 297 946

## Description

### Field of the Invention

The invention relates to devices for delivery of active agents to a subject, more specifically to devices for delivery of active agents into the lungs of a subject, such as inhaler devices.

### Background

There are a number of active agents that are useful in treating various diseases or conditions that need to be administered to the subject via the lungs, i.e. they are pulmonary delivered active agents. Such pulmonary delivered active agents typically use devices that allow the subject to inhale the active agent directly into the lungs, such as inhalers.

Typically, inhalers in the art are designed to be used multiple times to minimise waste and to provide the subject with a single delivery system that they can carry with them to provide a reliable delivery system for when the subject needs them. For example, it is important for subjects suffering from asthma to have a delivery system to hand whenever they may suffer from an asthma attack for delivering the necessary active agent quickly and efficiently.

However, such inhaler devices have suffered from the active agent and the carrier used to allow the active agent to be successfully delivered to the lungs of the subject agglomerating and requiring the active agent and carrier to be de-agglomerated prior to use. Furthermore, many devices are intended for multiple uses and so must contain multiple doses of active agent. For each use, the active agent must be accurately and reliably metered so that each dose is the same.

Solutions to these problems involve increasingly complicated devices including deagglomerating dispersion chambers to ensure that the active agent is suitably de-agglomerated and features to accurately meter the active agent for each dose, resulting in an increasingly bulky and less convenient device for the subject to carry and use.

Therefore, there is a need for improved inhaler devices that are convenient to carry for a subject and that are reliable.

Single use compact inhaler systems have been described in International patent application no. WO2019/008336 that provide significant advantages over conventional inhaler systems and include a membrane spanning a channel between two flexible substrates. WO2021191606A1 discloses another inhaler with two flexible substrates and a membrane.

However, there remain challenges for loading an active agent on a membrane in a way to allow the active agent to be retained on the membrane prior to use but to also allow the active agent to be released during use.

Accordingly, there remains a need for improved inhaler devices.

As a result, it is at least one object of the invention to provide an improved device for delivery of active agents to the lungs of a subject.

### Summary

The scope of the claimed invention is defined by the appended claims.

According to a first aspect there is provided an inhaler device comprising two flexible substrates and a membrane located between the two flexible substrates, each of the two flexible substrates comprising at least one deformable element, the two flexible substrates being connected at two opposing edges and unconnected at two further opposing edges, wherein the device is configured to move between a first configuration where the two flexible substrates are substantially flat and in contact with one another, and a second configuration where the two flexible substrates are flexed such that a channel is formed between the two flexible substrates having a first opening at one end of the channel and a second opening at an opposed end of the channel, wherein the membrane is configured to span the channel between the two flexible substrates when the device is in the second configuration, such that an active agent provided on the membrane may be inhaled by a user when the device is in the second configuration, wherein the membrane comprises a first portion facing the first opening and a second portion facing the second opening, the first portion being configured to releasably retain the active agent and the second portion being configured to prevent the passage of the active agent through the second portion.

The first portion of the membrane comprises a first plurality of apertures or pores, the second portion of the membrane comprises a second plurality of apertures or pores, and the apertures or pores of the first plurality of apertures or pores are larger than the apertures or pores of the second plurality of apertures or pores; or the first portion of the membrane and the second portion of the membrane comprise a mesh, and the mesh of the first portion of the membrane has larger gaps, holes or voids than the mesh of the second portion of the membrane.

Typically, the first opening of the channel may be an air outlet and the second opening of the channel may be an air inlet such that during use air is taken into the channel via the air inlet and inhaled out of the channel via the air outlet.

The unconnected two further opposing edges may be edges of the inhaler device when the inhaler device is in the first configuration. The unconnected two further opposing edges may be edges of the inhaler device when the inhaler device is in the second configuration. The first opening of the channel and the second opening of the channel may be adjacent or at the two opposing edges at which the two flexible substrates are unconnected. Accordingly, the channel may extend between the unconnected two opposing edges.

Accordingly, during use the active agent retained on or within the first portion of the membrane is released and inhaled by the user as air is inhaled through the channel from the second opening, through the membrane and out of the first opening.

The inventors have found that the device of the present aspect provides a simple way of delivering an active agent to the lungs of a subject, which is compact, mobile and easy to use.

As used herein, the term "span the channel" refers to the membrane extending across the channel to occlude at least a portion of the cross-section of the channel.

Further, it has been found that the provision of a membrane with a first portion that is configured to releasably retain the active agent allows the membrane to be readily loaded with active agent during manufacture whilst the second portion of the membrane prevents the active agent being lost through the membrane during manufacture.

Yet further, the provision of a membrane with a second portion that is configured to prevent the passage of the active agent through the second portion ensures that during use the active agent cannot be lost through the second opening if the user exhales into the inhaler device prior to inhalation.

The first portion may be at least 50% of the membrane. The first portion may be at least 60% of the membrane. The first portion may be at least 70% of the membrane. The first portion may be at least 80% of the membrane. At least 50% of the depth of the membrane may be the first portion of the membrane such that when viewed from side on at least 50% of the depth of the membrane is made up of the first portion. At least 60% of the depth of the membrane may be the first portion of the membrane such that when viewed from side on at least 60% of the depth of the membrane is made up of the first portion. At least 70% of the depth of the membrane may be the first portion of the membrane such that when viewed from side on at least 70% of the depth of the membrane is made up of the first portion. At least 80% of the depth of the membrane may be the first portion of the membrane such that when viewed from side on at least 80% of the depth of the membrane is made up of the first portion.

The membrane may be from about 50% to about 99% the first portion. The membrane may be from about 60% to about 99% the first portion. The membrane may be from about 70% to about 99% the first portion. The membrane may be from about 80% to about 99% the first portion. The membrane may be from about 50% to about 95% the first portion. The membrane may be from about 50% to about 90% the first portion. The membrane may be from about 50% to about 80% the first portion. The membrane may be from about 50% to about 70% the first portion.

The second portion may act as a backing layer provided on the first portion.

The membrane comprises a first major surface and an opposed second major surface. The first portion of the membrane may comprise the first major surface of the membrane. The second portion of the membrane may comprise the second major surface of the membrane.

The first portion of the membrane may be gas permeable to allow air to pass through the membrane during use. The second portion of the membrane may be gas permeable to allow air to pass through the membrane during use. Preferably, the first portion of the membrane and the second portion of the membrane are gas permeable to allow air to pass through the membrane during use.

The first portion of the membrane may be porous. The second portion of the membrane may be porous. Typically, the first portion of the membrane may be more porous than the second portion of the membrane. Accordingly, the first portion of the membrane may have a greater porosity than the second portion of the membrane.

Typically the membrane is a unitary membrane. The first portion and the second portion may be portions of the same membrane. Accordingly, the first portion and the second portion may abut or contact each other such that there is no space or gap between them. The first portion may occlude the second portion. Accordingly, the first portion and the second portion may completely overlap with one another. The first portion and the second portion may overlap one another for at least the majority of the extent of their surface. The first portion and the second portion may be different layers of material that have been laminated together, for example.

The active agent may comprise particles of active agent. Accordingly, the active agent may be a particulate active agent.

Typically, the second portion of the membrane may be impervious to the active agent.

The first portion of the membrane may comprise a first plurality of apertures or pores. The second portion of the membrane may comprise a second plurality of apertures or pores. The apertures or pores of the first plurality of apertures or pores may be larger than the apertures or pores of the second plurality of apertures or pores. The average size of the apertures or pores of the first plurality of apertures or pores may be larger than the average size of the apertures or pores of the second plurality of apertures or pores.

Typically, the first plurality of apertures or pores may allow particles of active agent to at least partially penetrate into the first portion of the membrane. Accordingly, at least a majority of first plurality of apertures or pores are significantly larger than the particles of active agent.

Typically, the second plurality of apertures or pores may prevent particles of active agent to penetrate or partially penetrate into the second portion of the membrane.

Particles of active agent that are typically used in inhaler devices have a mean diameter of less than 100 µm, less than 50 µm, less than 25 µm, less than 10 µm, or less than 5 µm. The particles may have a mean diameter of from 0.5 µm to 100 µm. The particles may have a mean diameter of from 0.5 µm to 50 µm. The particles may have a mean diameter of from 0.5 µm to 25 µm. The particles may have a mean diameter of from 0.5 µm to 10 µm. The particles may have a mean diameter of from 0.5 µm to 5 µm. The particles may have a mean diameter of from 1 µm to 100 µm. The particles may have a mean diameter of from 1 µm to 50 µm. The particles may have a mean diameter of from 1 µm to 25 µm. The particles may have a mean diameter of from 1 µm to 10 µm. The particles may have a mean diameter of from 1 µm to 5 µm.

Accordingly, the first plurality of apertures or pores may be larger than 100 µm in at least a major dimension, larger than 50 µm in at least a major dimension, larger than 25 µm in at least a major dimension larger than 10 µm in at least a major dimension, or larger than 5 µm in at least a major dimension when the particles of active agent have a mean diameter less than 100 µm, less than 50 µm, less than 25 µm, less than 10 µm, or less than 5 µm respectively.

In some embodiments, the first portion of the membrane may be substantially continuous and provide a substantially continuous surface upon which an active agent may be retained prior to use. The first portion of the membrane may have pores that allow air to pass across the first portion of the membrane but that are small enough to prevent particles of active agent to pass through.

In some embodiments, the second portion of the membrane may be substantially continuous and provide a substantially continuous surface upon which an active agent may be mounted. For example, the second portion of the membrane may have pores that allow air to pass across the second portion of the membrane but that are small enough to prevent particles of active agent to pass through.

In some embodiments the first portion of the membrane may comprise a monolithic material. The monolithic material may comprise a plurality of holes or apertures. The plurality of holes or apertures may be arranged on the first portion in an ordered manner. For example, the plurality of holes or apertures may be arranged in an array. The plurality of holes or apertures may be arranged in the first portion in a disordered manner.

In some embodiments the second portion of the membrane may comprise a monolithic material. The monolithic material may comprise a plurality of holes or apertures. The plurality of holes or apertures may be arranged on the first portion in an ordered manner. For example, the plurality of holes or apertures may be arranged in an array. The plurality of holes or apertures may be arranged in the first portion in a disordered manner. The holes or apertures of the plurality of holes or apertures may be significantly smaller than the diameter of the particles of active agents to be retained on or in the membrane.

In some embodiments, the first portion of the membrane may comprise a mesh. The mesh may comprise a network of fibres. The network of fibres may be woven together to form the mesh. The network of fibres may be connected at nodes to form the mesh. Particles of active agent may be adhered to the surface of the fibres of the mesh. The particles of active agent may be bound or loosely attached to the first portion of the membrane by an electrostatic charge. The electric charge applied to the first portion of the membrane and/or the particles of active agent may be adjusted to ensure that the particles of active agent is retained on the first portion of the membrane prior to use but is still lifted from the first portion of the membrane during use.

In some embodiments, the second portion of the membrane may comprise a mesh. The mesh may comprise a network of fibres. The network of fibres may be woven together to form the mesh. The network of fibres may be connected at nodes to form the mesh.

In embodiments where the first and second portions of the membrane comprise a mesh, the mesh of the first portion may have larger gaps, holes or voids than the mesh of the second portion. The mesh of the first portion may allow particles of active agent to permeate or penetrate the mesh such that the particles of active agent may be distributed within the mesh of the first portion. In contrast, the mesh of the second portion may have smaller gaps, holes or voids that are generally significantly smaller than the particles of active agent such that the mesh of the second portion is substantially impervious to the particles of active agent. Accordingly, the membrane may be configured to retain and to release particles of active agent whilst also ensuring that the membrane can be loaded with particles of active agent more reliably during manufacture.

In some embodiments, the first portion of the membrane may comprise a mesh and the second portion of the membrane may be substantially continuous.

The first portion may comprise foamed material. The foamed material may be an open cell foamed material. An open cell foamed material may comprise large voids connected together. The first portion may comprise strands of material that may form a scaffold around the voids.

For the avoidance of doubt, any embodiment of the first portion of the membrane may be combined with any embodiment of the second portion of the membrane as described above.

The membrane may comprise a polymer. For example, the membrane may comprise polytetrafluoroethylene (PTFE), expanded PTFE (ePTFE), polypropylene, polyethylene, polyurethane, poly-lactic acid, poly-glycolic acid, poly-caprolactone, poly(dioxanone), or a co-polymer thereof. The membrane may comprise polyethylene or polypropylene.

The first portion of the membrane may comprise the same material as the second portion of the membrane. The first portion of the membrane may be a more porous portion of the membrane and the second portion of the membrane may be a less porous portion of the membrane. Accordingly, the porosity of the first portion of the membrane may be greater than the porosity of the second portion of the membrane. The first portion of the membrane may be a less dense portion of the membrane and the second portion of the membrane may be a more dense portion of the membrane. Accordingly, the density of the first portion of the membrane may be less than the density of the second portion of the membrane.

The first portion of the membrane may comprise a different material than the material of the second portion of the membrane.

The first portion of the membrane may comprise a coating. The coating may change the surface properties of the first portion of the membrane. The coating may enhance one or more surface properties of the first portion of the membrane. The coating may make the surface of the first portion of the membrane more hydrophobic. The coating may make the surface of the first portion of the membrane more hydrophilic. The coating may change the ability of the surface of the first portion of the membrane to retain charge. Accordingly, the coating may make the surface of the first portion of the membrane more charged or less charged.

The coating may comprise an excipient. The coating may comprise one or more of excipients selected from the group: magnesium stearate; cellulose including microcrystalline cellulose, modified cellulose, such as hydroxylalkyl cellulose including hydroxypropyl cellulose and hydroxypropyl methyl cellulose (HPMC), alkyl cellulose such as methylcellulose, ethylcellulose or propylcellulose and carboxyalkyl celluloses such as carboxymethyl cellulose (CMC) and croscarmellose; starch; modified starches such as sodium starch glycolate and pregelatinized starch; silicone/titanium dioxide; stearic acid and salts of stearate such as calcium stearate; gelatin; talc; sucrose; polyvinylpyrrolidone (povidone) and crosslinked polyvinyl N-pyrrolidone (crospovidone); calcium phosphate; shellac and glazes.

In some embodiments the membrane may be a laminated membrane comprising two or more layers laminated together. The first portion of the membrane may correspond to one or more layers of the laminated membrane. The second portion of the membrane may correspond to one or more layers of the laminated membrane. Typically, the first portion corresponds to different layers of the laminated membrane to the layers of the second portion.

In some embodiments the membrane comprises a first membrane and a second membrane located adjacent to one another or in contact with one another. The first membrane may be secured to the second membrane directly. For example, the first membrane may be adhered, coupled or otherwise secured to at least a portion of the second membrane. The first membrane may be secured to the second membrane indirectly via a support or via one or both of the two flexible substrates such that the first membrane is not directly adhered, coupled or otherwise secured to the second membrane. Accordingly, the first portion of the membrane may be the first membrane and the second portion of the membrane may be the second membrane. Typically, the membrane does not comprise a space or gap between the first membrane and the second membrane.

In embodiments where the membrane is substantially continuous, the membrane may span only portions of the cross-section of the channel to ensure that a sufficient air flow may be created through the channel during use. Accordingly, there may be gaps in the cross-section of the channel that allow an increased airflow through the channel.

The membrane may be planar, or substantially planar. Alternatively, the membrane may comprise an indented portion. In embodiments where the membrane comprises an indented portion, a majority of the active agent on the membrane may be located within the indented portion. Accordingly, the indented portion may extend away from the outlet of the device, and towards the inlet of the device. In some embodiments, during use, the indented portion pay be everted when a user breathes in through the device. Accordingly, active agent retained within the indented portion may be propelled in the direction of airflow. The membrane may span and occlude the entire cross-section of the channel when the device is in the second configuration. Typically, the membrane spans the channel between the opposed open edges of the flexible substrates. The membrane may span or occlude a portion of the channel when the device is in the second configuration. As a result there may be portions of the channel where air can pass through the channel without passing through the membrane, and portions of the channel where air must pass through the membrane.

Typically, the membrane may be flexible and may be folded or collapsed when the device is in the first configuration.

The membrane may be configured to be compressed or condensed when the inhaler device is in the first configuration. The membrane may be configured to expand when the inhaler device moves from the first configuration to the second configuration. Expansion of the membrane may bias the device to the second configuration.

During manufacture active agent may be become trapped within the first portion of the membrane when the membrane is compressed. Accordingly, during use when the device is moved form the first configuration to the second configuration the membrane may be expanded and the active agent may become free or available to pass out of the first portion of the membrane when the user inhales through the inhaler device.

Typically, an active agent is located on the membrane or within the membrane. The active agent may be on the surface of the membrane. For example, the active agent may be in particulate form and the particles may be attached to the surface of the membrane. The active agent may be loosely attached to the surface of the membrane such that when air passes through the membrane during use, the active agent is dislodged from the membrane and becomes airborne. The active agent may be bound or loosely attached to the membrane by an electrostatic charge. The electric charge applied to the membrane and/or active agent may be adjusted to ensure that the active agent is retained on the membrane prior to use, but is still lifted from the membrane during use. As a result, the active agent may be readily inhaled by a subject into their lungs.

The active agent may be provided between the first portion and the second portion. The first portion may be configured to releasably retain the active agent between the first portion and the second portion prior to use. During use the active agent may pass through the first portion to the first opening. Accordingly, the first portion may act as a filter to ensure that large agglomerations of active agent are not inhaled by the user during use. The first portion may be connected to the second portion around the periphery of the membrane. The active agent may be provided between the first portion and the second portion away from the periphery of the membrane. The first portion may be adhered to the second portion around the periphery of the membrane. The periphery of the membrane may be adjacent to the wall of the channel.

In some embodiments, the particles of active agent may also comprise a carrier, vehicle or excipient. The carrier, vehicle or excipient may help prevent the particles from aggregating whilst the device is in the first configuration before use. The carrier, vehicle or excipient may enhance the ability of the or each active agent to become airborne when air passes through the channel of the device when the subject inhales, for example. The carrier, vehicle or excipient may prevent the particles from aggregating on the membrane.

Typically, the active agent on the membrane is sufficient for a user to receive one full dose of the active agent when they inhale through the device. Accordingly, the amount of active agent on the membrane may correspond to a single full dose. In some embodiments, when a user inhales through the device, some active agent may remain on the membrane. Therefore, the amount of active agent on the membrane may correspond to more than a single full dose, such that the amount of active agent that is actually inhaled by the user is a single full dose.

The membrane may be mounted within the channel on a support. The support may comprise a gas impermeable material that occludes the channel and comprise at least one aperture. The membrane may be mounted within the at least one aperture. Accordingly, the air flow through the channel may be constricted by the aperture within the support to thereby increase the rate of air flow through the membrane (namely, a venturi effect), thereby increasing the force exerted by the air flow on the active agent on the membrane to lift the active agent from the membrane and into the air flow.

In some embodiments, the support may comprise at least two apertures and a membrane may be supported across each aperture. Accordingly, a first membrane may be supported within a first aperture, and a second membrane may be supported within a second aperture. The first membrane may be provided with a first active agent. The second membrane may be provided with a second active agent. Therefore, the device may be configured to deliver two active agents at the same time to a user when the user inhales through the device. The first active agent may be provided in a first unit dose. The second active agent may be provided in a second unit dose. The first unit dose may be different to the second unit dose. The first unit dose may be the same as the second unit dose.

Alternatively, the first membrane may be provided with a first active agent and the second membrane may be provided with the first active agent. Accordingly, the device may provide a greater dose of the first active agent than devices with a single membrane.

The support may occlude the channel when the device is in the first configuration. The support may adopt a flexed or folded or otherwise reversibly collapsed configuration when the device is in the first configuration. When the device is moved to the second configuration, the support may open out to span and occlude the channel of the device. Typically, the support may open out to an open configuration and the support may not open any further. Accordingly, the support may ensure that the device may not be moved beyond the second configuration by a user, thereby ensuring that the optimum air flow is achieved by the device when the user inhales through the device in the second configuration.

The support may comprise at least one fold such that when the device is in the first configuration the support is folded about the at least one fold, and when the device is in the second configuration the support is extended and substantially unfolded. The at least one fold may extend across at least a portion of the membrane mounted on the support.

The support may comprise at least two folds. Each of the at least two folds may allow the support to fold in opposing directions. For example, a first fold may allow the support to be folded in a first direction, and a second fold may allow the support to be folded in an opposed second direction. When the device is in the first configuration the support may be folded about the at least two folds, and when the device is in the second configuration the support may be extended and substantially unfolded. One or more of the at least two folds may extend across at least a portion of the membrane mounted on the support.

The support may comprise at least three folds. Each of the at least three folds may allow the support to fold in opposing directions. For example, a first fold may allow the support to be folded in a first direction, a second fold may allow the support to be folded in an opposed second direction, and a third fold may allow the support to be folded in the first direction. Accordingly, the support may be folded in a concertina-type fashion. One or more of the at least three folds may extend across at least a portion of the membrane mounted on the support. The at least three folds may divide the support into at least four portions. The support may be divided into two central portions and two peripheral portions. The membrane may be mounted across an aperture in one of the four portions. The membrane may be mounted across an aperture in two of the four portions. The membrane may be mounted across the two central portions. The support may be mounted to the first flexible substrate by a first peripheral portion. The support may be mounted to the second flexible substrate by a second peripheral portion.

The support may comprise at least four folds. Each of the at least four folds may allow the support to fold in opposing directions. For example, a first fold may allow the support to be folded in a first direction, a second fold may allow the support to be folded in an opposed second direction, a third fold may allow the support to be folded in the first direction and a fourth fold may allow the support to be folded in the opposed second direction. Accordingly, the support may be folded in a concertina-type fashion. One or more of the at least four folds may extend across at least a portion of the membrane mounted on the support. Alternatively, the membrane may be located between two adjacent folds. The at least four folds may divide the support into at least five portions. The support may be divided into three central portions and two peripheral portions. The three central portions may comprise a middle portion in between two adjacent portions. The membrane may be mounted across an aperture in one of the four portions. The membrane may be mounted across two of the four portions. The membrane may be mounted across an aperture in the middle portion. The support may be mounted to the first flexible substrate by a first peripheral portion. The support may be mounted to the second flexible substrate by a second peripheral portion.

The support may comprise or form an enclosure when folded. The active agent may be located on the side of the membrane that is on the inside of enclosure of the folded support when the device is in the first configuration. Accordingly, the active agent may be protected within the enclosure prior to use. In embodiments where the support comprises at least four folds, the membrane may be located within the enclosure when the support is folded.

The support may comprise a seal adjacent to the edge of the enclosure when the support is folded. The seal may protect the active agent when the device is in the first configuration.

The support may be located in the middle of the channel such that the support is substantially equidistant from the first opening and the second opening. The support may be located adjacent to or closest to the first opening. The support may be located adjacent to or closest to the second opening.

Typically, the membrane is configured to ensure that during use when a subject inhales at one of the openings of the channel the air flow through the device is sufficient to dislodge a sufficient amount of the active agent or particles comprising the active agent from the membrane into the lungs of the subject to provide the dose of active agent required.

Preferably, the active agent is effective when delivered to the lungs of the subject. Therefore, the device of the present aspect is suitable for use for delivery of any active agent that may be delivered to the lungs of a subject.

Typically, the active agent is provided as a dry powder. The dry powder may comprise particles. The particles may comprise the active agent. The particles may comprise a carrier.

The active agent may be a bronchodilator. For example, the active agent may be salbutamol, salmeterol, formoterol, Ventolin, or other such active agent.

The active agent may be a vaccine. For example, the active agent may be an inhalable vaccine against diseases such as cholera, diphtheria, anthrax, tetanus, hepatitis A or B, influenza, measles, meningitis, polio, rabies, pneumonia, rotavirus, smallpox, typhoid, yellow fever, corona virus etc.

The active agent may treat pain. For example, the active agent may be an inhalable form of tramadol, gabapentin, Vicodin (registered trade mark), ibuprofen, acetaminophen, hydrocodone, naproxen, methadone, codeine, hydroxyzine, paracetamol, aspirin, etc.

The active agent may be used to treat diabetes. For example, the active agent may be an inhalable form of insulin, canagliflozin, alogliptin benzoate, dapaglifozin, empagliflozin, ranibizumab, duglaglutide, pioglitazone hydrochloride and glimepiride etc.

The active agent may be used to treat or prevent migraine. The active agent may be a triptan (or 5HT agonists) such as Almotriptan (such as Almogran^{™}), Eletriptan (such as Relpax^{™}), Frovatriptan (such as Migard^{™}), Naratritan (such as Naramig^{™}), Rizatriptan (such as Maxalt^{™}), Sumatriptan (such as lmigran^{™}), Zolmitriptan (such as Zomig^{™}), metoclopramide, rimegepant, lasmiditan, or similar.

The active agent may be a hormone, such as an inhalable form of oxytocin or similar. The active agent may prevent postpartum haemorrhage.

The active agent may be used to treat sexual health disorders. For example, the active agent may be an inhalable form of sildenafil, tadalafil or vardenafil.

The active agent may be used to treat nausea. For example, the active agent may be an inhalable form of ondansetron, domperidone, dolasetron, or dronabinol.

The active agent may be used to treat allergies. For example, the active agent may be an inhalable form of loratadine, cetirizine, chlorphenamine, diphenhydramine, or fexofenadine.

The active agent may be used to treat epilepsy. For example, the active agent may be an inhalable form of levetiracetam, lacosamide, or lamotrigine.

The active agent may be used to treat asthma.

The active agent may be used to treat multiple sclerosis and/or related symptoms. For example, the active agent may be an inhalable form of teriflunomide, or fingolimod.

The active agent may be used to treat Parkinson's disease. For example, the active agent may be an inhalable form of pramipexole, rotigotine, ropinirole, carbidopa-levodopa.

The active agent may be used to treat motor neurone disease (MND) and/or related symptoms. For example, the active agent may be an inhalable form of riluzole.

The active agent may be used to treat depression. For example, the active agent may be a psychoactive agent such as a cannabinoid, a selective serotonin reuptake inhibitors (SSRI).

The active agent may be used to treat infection. The active agent may be used to treat a viral infection. The active agent may be used to treat a bacterial infection.

The active agent may be a vitamin, a dietary supplement, a probiotic, or a natural stimulant such as caffeine, or a natural relaxant such as chamomile extract, or a herbal remedy. The active agent may be any other non-medical, inhalable agent that can be manufactured as an inhalable dry powder.

In some embodiments the at least one deformable element of each flexible substrate may bias the device into a second configuration where a first opening of the channel formed between the two flexible substrates is substantially regular in shape, and a second opening of the channel formed between the two flexible substrates is substantially irregular in shape.

A substantially regular shape may be a shape that has an aspect ratio that is from approximately 3:1 to 1:3 or from approximately 3:2 to 2:3.

A substantially irregular shape may be a shape that has an aspect ratio that is greater than approximately 3:1 or less than approximately 1:3 or is greater than approximately 3:2 or less than approximately 2:3.

As used herein, an aspect ratio of 3:1 has a height of 3 to a width of 1 or a ratio thereof.

The first opening of the channel may be polygonal. The first opening of the channel may be substantially rectangular. For example, the first opening of the channel may be substantially oblong or square. The first opening of the channel may be substantially rectangular with curved or rounded corners.

The second opening of the channel may be comprise at least two opposed curved sides. The second opening of the channel may be biconvex. An opening that is biconvex is convex on both sides or surfaces of the opening.

Accordingly, in some embodiments the at least one deformable element of each flexible substrate may bias the device into a second configuration where the first opening of the channel formed between the two flexible substrates is substantially rectangular, and the second opening of the channel formed between the two flexible substrates is substantially biconvex.

Typically, the two flexible substrates are the same shape. In some embodiments the two flexible substrates may be rectangular. In some embodiments the two flexible substrates may be square. Alternatively, in other embodiments the two flexible substrates may be oblong. It will be appreciated by the person skilled in the art that alternative shapes of the two flexible substrates are included within the scope of the present disclosure, as long as the two flexible substrates are connected at two opposing edges and can move between the first configuration and the second configuration. For example, the two flexible substrates may be trapezoidal, hexagonal, octagonal or similar. In another example, the two opposed edges that are not connected may be curved.

Typically, the two flexible substrates are uniform or substantially uniform substrates that may be flexed to move from the first configuration to the second configuration. However, at least one of the two flexible substrates may comprise two or more regions that have differing rigidity such that at least one of the two or more regions is more rigid and resistant to flexing, and at least one of the two or more regions is less rigid and less resistant to flexing. For example, one or both of the flexible substrates may comprise one or more flexible portions and one or more rigid portions. The one or more rigid portions may resist flexing and the one or more flexible portions may be readily flexed. As a result during use the one or more flexible region of at least one of the two flexible substrates may flex to allow the device to move from the first configuration to the second configuration, and the one or more rigid region remains substantially planar. The flexible region may form a hinge in the flexible substrate. The flexible region may be shaped such that the device is biased towards the second configuration. In some embodiments, the or each flexible portion may correspond to an at least one deformable element,
The at least one deformable element of each flexible substrate may be a scored (i.e. marked or cut) line along at least a portion of the flexible substrate. The at least one deformable element of each flexible substrate may be a flexible line extending along at least a portion of the flexible substrate. The at least one deformable element of each flexible substrate may be a crease in the flexible substrate.

The at least one deformable element of each flexible substrate may be formed by a series of perforations in the flexible substrate.

The at least one deformable element of each flexible substrate may be formed by a portion of a flexible substrate that is thinner than the majority of the flexible substrate.

Each flexible substrate may comprise a first flexible layer and a second layer that is less flexible that the firs flexible layer. The second layer may comprise a plurality of portions. each portion in the plurality of portions may be at least partially separated from one another by a space or gap. The at least one deformable element of each flexible substrate may correspond to a space or gap between adjacent portions of the second layer. Alternatively, the or each portion in the plurality of portions may abut one another forming a fault line between adjacent portions. The at least one deformable element of each flexible substrate may correspond to the fault line between adjacent portions.

The at least one deformable element may act as a hinge such that when the device moves between the first configuration and the second configuration each flexible substrate bends about the at least one deformable element.

The at least one deformable element may comprise a first portion and a second portion. The first portion may be adjacent to the first opening of the channel when the device is in the second configuration, and the second portion may be adjacent to the second opening of the channel when the device is in the second configuration. The first portion may connect to the second portion part way along the length of the flexible substrate.

The first portion may be linear or straight. The first portion may be aligned with an axis of the device that runs along the length of the channel. The first portion may be oriented at an angle to the axis of the device. For example, the first portion may be oriented at an angle of 1° to 40° to the axis of the device. The first portion may be oriented at an angle of 1° to 25° to the axis of the device. Accordingly, the first portion may extend from the first opening of the flexible substrate towards the second opening of the flexible substrate.

The first portion may be curved.

The second portion may be linear or straight. The second portion may be oriented at an angle to the axis of the device that runs along the length of the channel.

The second portion may be curved. The second portion may curve towards one of the connected edges of the flexible substrate. The second portion may curve towards one or the corners of the flexible substrate. The second portion may curve to a corner of the flexible substrate.

In embodiments where the first portion and the second portion are curved, the first portion may curve at a rate that is less than the rate of the second portion. The first portion may curve at a rate that is greater than the rate of the second portion. Accordingly, the at least two deformable elements may curved at a first rate in the portion corresponding to the first portion, and may be curved at a second rate in the portion corresponding to the second portion.

Each flexible substrate may comprise at least two deformable elements. The at least two deformable elements may be symmetrically arranged on the flexible substrate. The plane of symmetry may run along the centre line or central axis of the flexible substrate that runs along the length of the flexible substrate. The plane of symmetry may be oriented at 90° to the major plane of the flexible substrate. Accordingly, the device may be symmetrical around at least one plane of symmetry.

Moving from the first configuration to the second configuration may impose sufficient forces on the flexible substrates that they bend around the at least one deformable element.

The at least one deformable element may be configured to lock the device in the second configuration once the device is moved from the first configuration to the second configuration. Accordingly, once the device is in the second configuration, the device may be maintained in the second configuration without the application of external force by the user.

The device may further comprise at least one locking formation. The at least one locking formation may be configured to lock the device in the second configuration. The at least one locking formation may be configured to lock the device in the second configuration when the device is moved from the first configuration to the second configuration.

The at least one locking formation may be formed by at least a portion of the at least one deformable element of the first flexible substrate and the second flexible substrate. In embodiments where the at least one deformable element comprises a curved second portion, the at least one locking formation may be formed by at least a portion of the curved second portion of the at least one deformable element of the first flexible substrate and at least a portion of the curved second portion of the at least one deformable element of the second flexible substrate. The locking formation may be an indentation in the body of the device that is formed when the device is moved from the first configuration to the second configuration. The indentation may lock the device into the second configuration as the indentation is formed.

In some embodiments, the at least one locking formation may generate an audible cue when the at least one locking formation moves into a locking position. The at least one locking formation may generate a popping noise, for example.

The flexible substrates may comprise card or cardboard. The flexible substrates may comprise a polymer or plastic. The polymer or plastic may be a thermoplastic. The polymer or plastic may be a thermoplastic selected from the group consisting polypropylene (PP), polyethylene terephthalate (PET), and high-density polyethylene (HDPE). The flexible substrates may comprise a combination of card and polymer or plastic, such as a card or cardboard substrate with a polymer or plastic coating. The polymer or plastic coating may be provided on the external surface of the flexible substrates. The polymer or plastic coating may be provided on the internal surface of the flexible substrates. The polymer or plastic coating may be provided on both the internal surface and the external surface of the flexible substrates.

One or both of the two flexible substrates may be degradable. One or both of the two flexible substrates may be biodegradable. One or both of the two flexible substrates may comprise a biopolymer. The biopolymer may a polypeptide, a nucleic acid or a polysaccharide. For example, the device may degrade when contacted to water, exposure to light or in the presence of bacteria or similar.

The membrane and an active agent thereon may be protected from moisture, light, oxygen and contamination. The membrane may be retained within a protective pocket between the two flexible substrates. The protective pocket may open, exposing the membrane and active agent thereon when the device is moved from the first configuration to the second configuration. The protective pocket may comprise a material that is resistant to water, oxygen and/or light. For example, the protective pocket may comprise a metallic foil, such as aluminium, or a polymer or plastic film.

The membrane may be occluded by a barrier material on at least one side of the membrane when the device is in at least the first configuration. The barrier material may form a barrier membrane or a barrier film across the membrane. The barrier material and the membrane may retain the active agent between them when the device is in the first configuration. Accordingly, the active agent may be protected from the environment by the membrane and the barrier material. The barrier material may be located adjacent to the membrane. The barrier material may be located on the side of the membrane facing the first opening of the device. Accordingly, during use, air may be inhaled through the device from the second opening, through the membrane, past the barrier material to the first opening.

The barrier material may be located on each side of the membrane to form a first barrier and a second barrier such that the membrane and active agent are both retained between the first barrier and the second barrier.

The barrier material may be attached to one or more of the flexible substrates. The barrier material may be attached to the membrane. The barrier material may be attached to a substrate upon or within which the membrane is supported.

The barrier material may be breached or detached to expose the active agent when the device is moved from the first configuration to the second configuration.

The barrier material may be breached or detached to expose the active agent when a user inhales through the channel of the device when the device is in the second configuration. The barrier material may burst to expose the active agent when a user inhales through the channel of the device when the device is in the second configuration.

The barrier material may be located either side of the membrane to form a protective pocket within which the membrane and active agent are retained when the device is in the first configuration. The barrier material of the protective pocket may be broken or breached or detached when the device is moved from the first configuration to the second configuration. The barrier material of the protective pocket may be broken or breached or detached when a user inhales through the device.

The barrier material may comprise a material that is resistant to water, oxygen and/or light. For example, the barrier material may comprise a metallic foil, such as aluminium, or a polymer or plastic film.

At least a portion of at least one side of one or both of the two flexible substrates may comprise a metallic coating. For example, at least a portion of the interior surfaces of the two flexible substrates may comprise a metallic coating. The metallic coating may be a foil coating or similar. The metallic coating may comprise aluminium, copper or tin, for example.

In some embodiments, the metallic coating covers substantially the entire interior surface of both of the two flexible substrates. In some embodiments, the metallic coating covers a portion of the interior surface of both of the two flexible substrates. The portion may be adjacent to one of the unconnected opposing ends of both of the two flexible substrates. The portion may be part way between the two opposing unconnected ends of both of the two flexible substrates.

Typically, the metallic coating is located such that the membrane is at least partially covered by the metallic coating when the device is in the first configuration and the two flexible substrates are substantially flat. In some embodiments where the two flexible substrates comprise a metallic coating, the membrane is contained within an envelope or similar where the envelope comprises the metallic coating of the two flexible substrates. The envelope may be sealed such that the membrane is sealed within the envelope. Accordingly, the active agent provided on the membrane may be protected from moisture, oxygen, light and contamination.

The envelope may be sealed adjacent to the membrane. In embodiments where substantially the entire interior surface of both of the two flexible substrates is covered by the metallic coating, the envelope formed by the metallic coatings may be sealed adjacent to one or both of the unconnected opposing ends of the two flexible substrates.

The device may be a single use device. That is, the membrane between the two flexible substrates may comprise a single dose of active agent, and once the device has been used by a subject, the device may be discarded, and replaced by a new device.

In embodiments where the two flexible substrates are degradable, the discarded devices may degrade when contacted with water etc., thereby leaving minimal waste. In embodiments where the device comprises card or polymer or plastic, the device may be recycled to minimise waste.

The device may be protected from moisture. The device may be stored in waterproof packaging before use. The device may comprise one or more seals. The device may comprise one or more seals such that the membrane is sealed within the device and is thereby protected from moisture. For example, the device may comprise a seal adjacent to each opening. In another example, the device may comprise a seal either side of the membrane. During use, the action of moving the device from the first position to the second position may break the or each seal such that the membrane and any active agent mounted thereon is exposed.

In embodiments where the two flexible substrates comprise a metallic coating, the device may comprise seals at each opening of the channel and seals at either side of the metallic coating.

The device may comprise one or more reinforcing elements. The or each reinforcing element may bias the device toward the second configuration. The or each reinforcing element may provide a biasing force that is insufficient to move the device from the first configuration to the second configuration, and complements the force applied by a user to open or move the device from the first configuration to the second configuration.

The device may comprise one or more reinforcing elements in a central region of the device. The device may comprise one or more reinforcing elements adjacent to one or more of the openings of the channel. The reinforcing elements may allow the device to more readily move from the first configuration to the second configuration when a threshold pressure is applied by the user to the two opposing connected edges.

The or each reinforcing element may extend across one or both flexible substrates. The or each reinforcing element may extend across the width of the channel. That is, the or each reinforcing element may extend between the connected edges of the or each flexible substrate.

The or each reinforcing element may be shaped to promote opening of the channel when the two flexible substrates are flexed. For example, the or each reinforcing element may be curved or bent such that when pressure is applied to the connected opposing edges, the device is biased toward the second configuration.

The device may be dimensioned to fit within a user's hand. In some embodiments the device may be dimensioned to fit within a user's wallet or purse. For example, the device may be the size of a typical credit card or similar (i.e. generally planar having two major dimensions approximately 80-90 mm by 50-60 mm, such as 86mm by 54mm, for example). As a result, the device may be retained by a user in their wallet or purse to ensure that the device is readily to hand should the user require a dose of the active agent.

In some embodiments, the channel is configured to optimise air flow through the channel.

The cross-section of the channel may decrease from the air inlet to the air outlet, such that the air flow through the channel is accelerated from the air inlet to the air outlet.

The cross-section of the channel may be reduced in a portion of the channel. The cross-section of the channel may be reduced in a portion of the channel between the air inlet and the air outlet.

Typically, the height of the channel in one dimension may be greater at the second opening than at the first opening. The width of the channel may be greater at the first opening than at the second opening. For example, the height of the channel when viewed from the side may be greater at the second opening that at the first opening, and the width of the channel when viewed from above may be greater at the first opening than at the second opening.

In at least one embodiment, the cross-section of the channel may have a "trumpet-like" shape
The channel may comprise a first portion and a second portion. In at least one dimension, the cross-section of the channel within the first portion may be larger than the cross-section of the second portion. In at least one dimension, the cross-section of the channel within the first portion may be smaller than the cross-section of the second portion. Accordingly, where the rate of airflow is constant through the channel, the air must travel more quickly through the second portion compared to the first portion.

The second portion may comprise an aperture that constricts the channel. The membrane may span the aperture such that air flowing through the second portion must flow through the membrane. Accordingly, the air is moving faster through the membrane than through the first portion of the channel, thereby imposing a greater force on the active agent present on the membrane to lift that active agent into the air flow.

In some embodiments the channel may extend across the full width of the two flexible substrates. In alternative embodiments, the channel may extend across only a portion of the full width of the two flexible substrates. The two flexible substrates may comprise features such as creases or more pliant portions that define the width of the channel. For example, the two flexible substrates may comprise creases that run along the length of the two flexible substrates and that are spaced from the opposed connected edges of the two flexible substrates. During use, when the user moves the device from the first configuration to the second configuration, the channel is formed between the two creases, and the two flexible substrates remain substantially flat between the crease and associated connected edge either side of the channel.

The device may comprise an element that restricts the maximum extent to which the two flexible substrates can be flexed to move the device to the second configuration. For example, the device may comprise one or more connectors attached to each of the two flexible substrates such that when the device is in the second configuration, the separation of the two flexible substrates is determined by the length of the or each connector. The one or more connectors may be attached to the interior surface of each of the two flexible substrates within the channel. The one or more connectors may be attached to the exterior surface of each of the two flexible substrates. Alternatively, the device may comprise one or more connectors that extend across the width of the device that restrict the maximum extent to which at least one of the two flexible substrates may be bent. The device may comprise a rigid tertiary structure or a triangular lock fold that define the maximum extent the channel of the device may open.

The device may comprise a mouthpiece adjacent to or at the air outlet. Accordingly, during use the user may contact their mouth to the mouthpiece when the device is in the second configuration and then inhale through the mouthpiece. The mouthpiece may be the air outlet. The mouthpiece may be configured to provide support to the lips of a user during use.

The invention extends in a second aspect to a method of using a device according to the first aspect, the method comprising the steps:
(i) providing a device according to the first aspect;
(ii) applying pressure to the two opposed connected edges of the two flexible substrates of the device to thereby move the device from the first configuration, to the second configuration; and
(iii) inhaling adjacent to an opening of the device in the second configuration to thereby inhale an active agent from the membrane of the device through the channel and into the lungs.

When the device is in the second configuration, the user may contact their mouth to an opening of the channel. Therefore, the user may inhale through the device in step (iii). A seal may be formed between the device and the mouth of the user. Accordingly, when the user inhales, all or substantially all air that passes into the user's mouth has passed through the channel of the device and thereby carries active agent from the membrane of the device.

The user may apply pressure to the two opposed edges of the two flexible substrates by squeezing those edges toward each other.

In embodiments where the device comprises seals, step (ii) typically breaks said seals to thereby expose the membrane of the device.

In embodiments where the device is provided in packaging, the device is typically removed from said packaging prior to step (ii).

Once the user has inhaled through the device, the device may be discarded.

In some embodiments, the device may lock when moved from the first configuration to the second configuration to thereby lock the device in the second configuration. Accordingly, the device may be retained in the second configuration without continued application of force by the user.

According to a third aspect there is provided an inhaler device comprising a membrane located within a channel having a first opening and an opposed second opening, wherein the membrane is configured to span the inhaler channel such that an active agent provided on the membrane may be inhaled by a user through the channel. The membrane may comprise a first portion facing the first opening and a second portion facing the second opening, the first portion being configured to releasably retain the active agent and the second portion being configured to prevent the passage of the active agent through the second portion.

The first portion of the membrane and the second portion of the membrane comprise a mesh, and the mesh of the first portion of the membrane has larger gaps, holes or voids than the mesh of the second portion of the membrane.

Typically, the first opening of the channel may be an air outlet and the second opening of the channel may be an air inlet such that during use air is taken into the channel via the air inlet and inhaled out of the channel via the air outlet.

As used herein, the term "span the channel" refers to the membrane extending across the channel to occlude at least a portion of the cross-section of the channel.

Further, it has been found that the provision of a membrane with a first portion that is configured to releasably retain the active agent allows the membrane to be readily loaded with active agent during manufacture whilst the second portion of the membrane prevents the active agent being lost through the membrane during manufacture.

Yet further, the provision of a membrane with a second portion that is configured to prevent the passage of the active agent through the second portion ensures that during use the active agent cannot be lost through the second opening if the user exhales into the inhaler device prior to inhalation.

The inhaler may comprise a body. The body may be tubular. Accordingly, the body may be a tube. The channel may correspond to the interior of the tubular body. The body may comprise a longitudinal axis that extends along the length of the body. The body may be flexible. The body may comprise a flexible portion. The flexible portion may be more flexible than the remainder of the body. The flexible portion may allow one of the first opening and the opposed second opening to be reoriented relative to the other. For example, the first opening may be reoriented relative to the second opening by bending the body in the flexible portion. Conversely, the second opening may be reoriented relative to the first opening by bending the body in the flexible portion.

The first opening may comprise a covering. The second opening may comprise a covering. The first opening and the second opening may comprise a covering. The covering of the first opening and/or second opening may occlude the first opening and/or second opening. The covering of the first opening and/or second opening may seal the first opening and/or second opening. In embodiments where the first opening and second opening are sealed by a cover, the interior of the inhaler may be sealed from the exterior of the inhaler by the covers.

Typically, prior to use, the covering of the first opening and/or second opening may be removed.

The membrane may be provided within the channel substantially mid-way between the first opening and the second opening. Accordingly, the membrane may be provided substantially in the middle of the channel between the first opening and the second opening. The membrane may be provided adjacent to the first opening. The membrane may be provided adjacent to the second opening. The membrane may be provided in a half of the channel adjacent to the first opening. The membrane may be provided in a half of the channel adjacent to the second opening.

In embodiments where the body comprises a flexible portion, the membrane may be provided within the channel in the flexible portion. The membrane may be provided within the channel in a portion of the channel that is not in the flexible portion.

Typically, during use a user may insert the first opening in their mouth inhale through the inhaler device to thereby deliver the active agent from the first portion of the membrane to the lungs of the user through their mouth. Alternatively, the user may insert the first opening in a nostril of their nose and sniff through the device to thereby inhale through their nose the active agent from the first potion of the membrane to their lungs or to the sinuses of the user.

In embodiments where the body comprises a flexible portion, the user may bend the inhaler device and insert the second opening into their mouth and the first opening in a nostril of their nose. The user may then blow through the device to thereby deliver the active agent from the first portion of the membrane to the lungs of the user through their nose or to the sinuses of the user.

The first portion of the membrane may be at least 50% of the membrane. The first portion of the membrane may be at least 60% of the membrane. The first portion of the membrane may be at least 70% of the membrane. The first portion of the membrane may be at least 80% of the membrane. At least 50% of the depth of the membrane may be the first portion of the membrane such that when viewed from side on at least 50% of the depth of the membrane is made up of the first portion. At least 60% of the depth of the membrane may be the first portion of the membrane such that when viewed from side on at least 60% of the depth of the membrane is made up of the first portion. At least 70% of the depth of the membrane may be the first portion of the membrane such that when viewed from side on at least 70% of the depth of the membrane is made up of the first portion. At least 80% of the depth of the membrane may be the first portion of the membrane such that when viewed from side on at least 80% of the depth of the membrane is made up of the first portion.

The membrane may be from about 50% to about 99% the first portion. The membrane may be from about 60% to about 99% the first portion. The membrane may be from about 70% to about 99% the first portion. The membrane may be from about 80% to about 99% the first portion. The membrane may be from about 50% to about 95% the first portion. The membrane may be from about 50% to about 90% the first portion. The membrane may be from about 50% to about 80% the first portion. The membrane may be from about 50% to about 70% the first portion.

The second portion may act as a backing layer provided on the first portion.

The membrane comprises a first major surface and an opposed second major surface. The first portion of the membrane may comprise the first major surface of the membrane. The second portion of the membrane may comprise the second major surface of the membrane.

The first portion of the membrane may be gas permeable to allow air to pass through the membrane during use. The second portion of the membrane may be gas permeable to allow air to pass through the membrane during use. Preferably, the first portion of the membrane and the second portion of the membrane are gas permeable to allow air to pass through the membrane during use.

The first portion of the membrane may be porous. The second portion of the membrane may be porous. Typically, the first portion of the membrane may be more porous than the second portion of the membrane. Accordingly, the first portion of the membrane may have a greater porosity than the second portion of the membrane.

Typically the membrane is a unitary membrane. The first portion and the second portion may be portions of the same membrane. Accordingly, the first portion and the second portion may abut or contact each other such that there is no space between them. The first portion may occlude the second portion. Accordingly, the first portion and the second portion may completely overlap with one another. The first portion and the second portion may overlap one another for at least the majority of the extent of their surface. The first portion and the second portion may be different layers of material that have been laminated together, for example.

The active agent may be provided between the first portion and the second portion. The first portion may be configured to releasably retain the active agent between the first portion and the second portion prior to use. During use the active agent may pass through the first portion to the first opening. Accordingly, the first portion may act as a filter to ensure that large agglomerations of active agent are not inhaled by the user during use. The first portion may be connected to the second portion around the periphery of the membrane. The active agent may be provided between the first portion and the second portion away from the periphery of the membrane. The first portion may be adhered to the second portion around the periphery of the membrane. The periphery of the membrane may be adjacent to the wall of the channel.

The active agent may comprise particles of active agent. Accordingly, the active agent may be a particulate active agent.

Typically, the second portion of the membrane may be impervious to the active agent.

The first portion of the membrane may comprise a first plurality of apertures or pores. The second portion of the membrane may comprise a second plurality of apertures or pores. The apertures or pores of the first plurality of apertures or pores may be larger than the apertures or pores of the second plurality of apertures or pores. The average size of the apertures or pores of the first plurality of apertures or pores may be larger than the average size of the apertures or pores of the second plurality of apertures or pores.

Typically, the first plurality of apertures or pores may allow particles of active agent to at least partially penetrate into the first portion of the membrane. Accordingly, at least a majority of first plurality of apertures or pores are significantly larger than the particles of active agent.

Typically, the second plurality of apertures or pores may prevent particles of active agent to penetrate or partially penetrate into the second portion of the membrane.

For the avoidance of doubt, features of the membrane of the first aspect are features of the membrane of third aspect and vice versa.

### Brief Description of the Figures

Embodiments of the present invention will now be described, by way of non-limiting example, with reference to the accompanying drawings.
**Figure 1****:** a schematic side view of a section of a device according to an embodiment A) before and B) during inhalation;
**Figure 2****:** a schematic side view of a section of a device according to an embodiment A) before and B) during inhalation;
**Figure 3****:** A magnified image of an open pore foamed material;
**Figure 4****:** A cross section of a membrane according to an embodiment;
**Figure 5****:** a device according to an embodiment showing the device A) in the first configuration and B) in the second configuration;
**Figure 6****:** a device according to an embodiment in the second configuration A) viewed from the front (outlet end) and B) viewed from behind (inlet end);
**Figure 7****:** a membrane support according to an embodiment A) in a flat configuration and B) in a folded configuration;
**Figure 8****:** a membrane support according to an embodiment A) in a flat configuration and B) in a folded configuration;
**Figure 9****:** a side view of a membrane support according to an embodiment in a folded configuration;
**Figure 10****:** a membrane support according to an embodiment A) in a flat configuration and B) in a folded configuration;
**Figure 11****:** a view of a device according to an embodiment from A) a top view, B) a side view, and C) a perspective view;
**Figure 12****:** a perspective view of a device according to an embodiment;
**Figure 13****:** a perspective view of a device according to an embodiment; and
**Figure 14****:** a schematic side view of a section of a device according to an embodiment A) before and B) during inhalation.

### Detailed Description

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

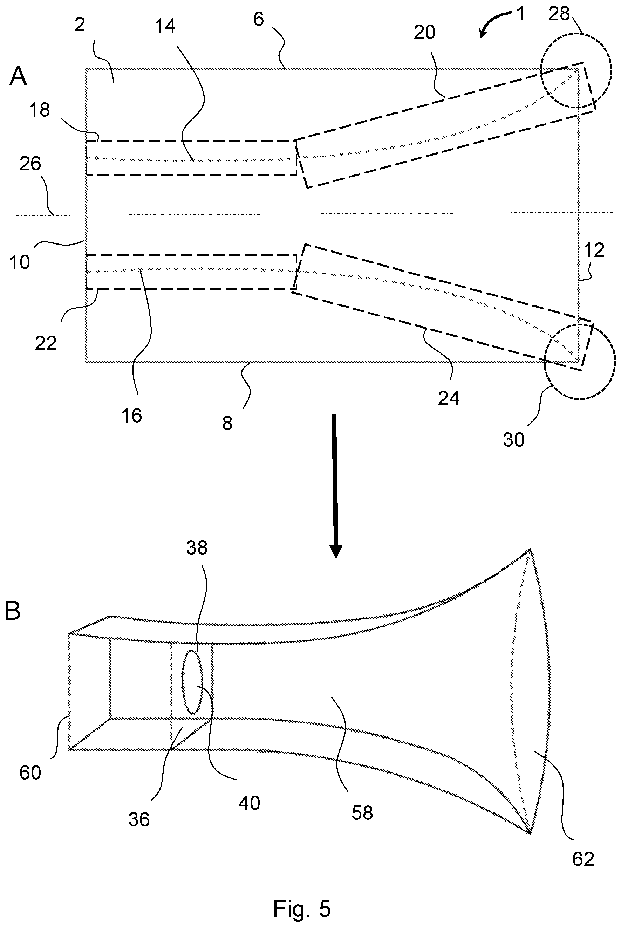

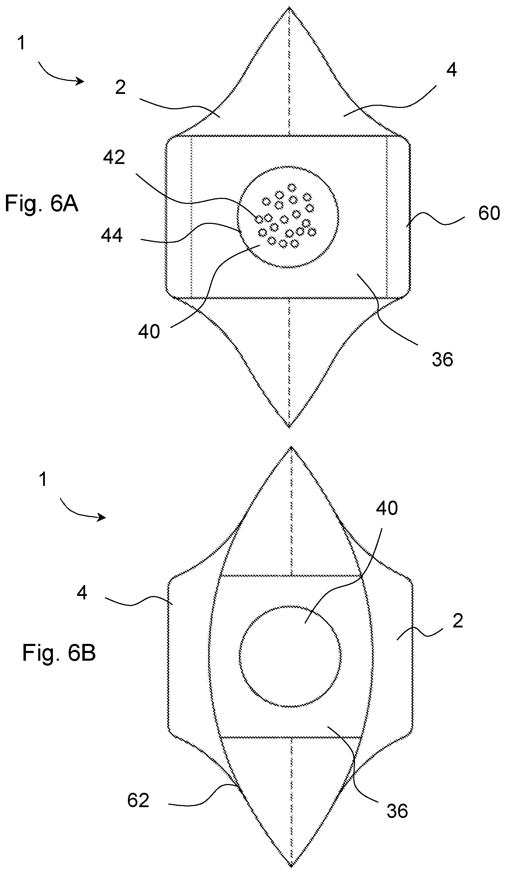

With reference to Figures 5 and 6, there is provided a device 1 comprising a first flexible substrate 2 and a second flexible substrate 4. The first flexible substrate 2 and the second flexible substrate 4 are rectangular. The first flexible substrate 2 and the second flexible substrate 4 are connected to one another along a first edge 6 and along a second edge 8 opposed to the first edge 6. The first flexible substrate 2 and the second flexible substrate 4 are not connected to one another along a third edge 10 and along a fourth edge 12 opposed to the third edge 8.

The first flexible substrate 2 comprises a first scored line 14 (acting as a first deformable element) and a second scored line 16 (acting as a second deformable element). The first scored line 14 comprises a linear portion 18 (acting as a first portion of the first deformable element) and a curved portion 20 (acting as a second portion of the first deformable element). The second scored line 16 comprises a linear portion 22 (acting as a first portion of the second deformable element) and a curved portion 24 (acting as a second portion of the second deformable element). The first scored line 14 and the second scored line 16 are arranged symmetrically about a central axis 26 running along the length of the first flexible substrate 2. The curved portion 24 of the first scored line 14 extends from the linear portion 22 to a first corner 28 of the first flexible substrate 2. The curved portion 24 of the second scored line 16 extends from the linear portion 22 of the second scored line 16 to a second corner 30 of the first flexible substrate 2.

The second flexible substrate 4 comprises a first scored line 32 and a second scored line 34 that correspond to the first scored line 14 and second scored line 16 of the first flexible substrate 2.

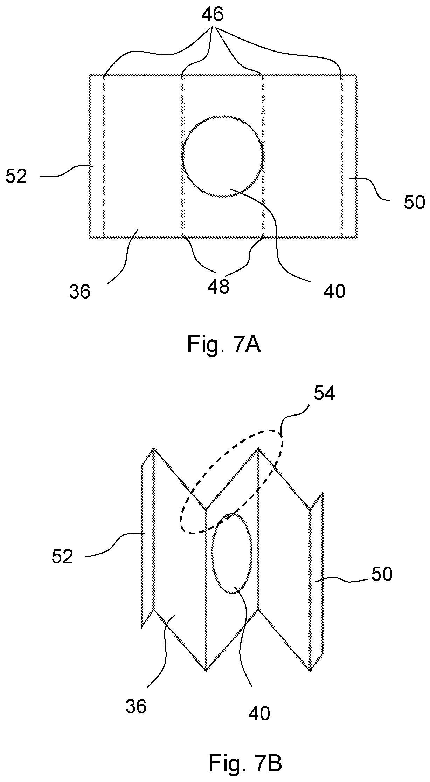

With reference to Figures 7A and 7B, the device 1 further comprises a support 36 located between the first flexible substrate 2 and the second flexible substrate 4. The support 36 comprises an aperture 38 that is spanned by a membrane 40. An inhalable form of an active agent 42 is supported on a first side 44 of the membrane 40. As shown in Figures 7A and 7B, the support 36 has four folds 46 such that the support 36 can be folded in a concertina-like fashion (see Figure 7B, for example). The membrane 40 is located centrally on the support 36 with a fold 48 either side of the membrane 40. The support 36 is further folded adjacent to the sides to provide a first attachment surface 50 and a second attachment surface 52 that is used to contact and be fixed to the first flexible substrate 2 and the second flexible substrate 4 respectively. Accordingly, when the support 36 is folded about the four folds 46 the membrane 40 is retained within a pocket 54 (corresponding to an enclosure) such that the active agent 42 supported on the membrane 40 is protected.

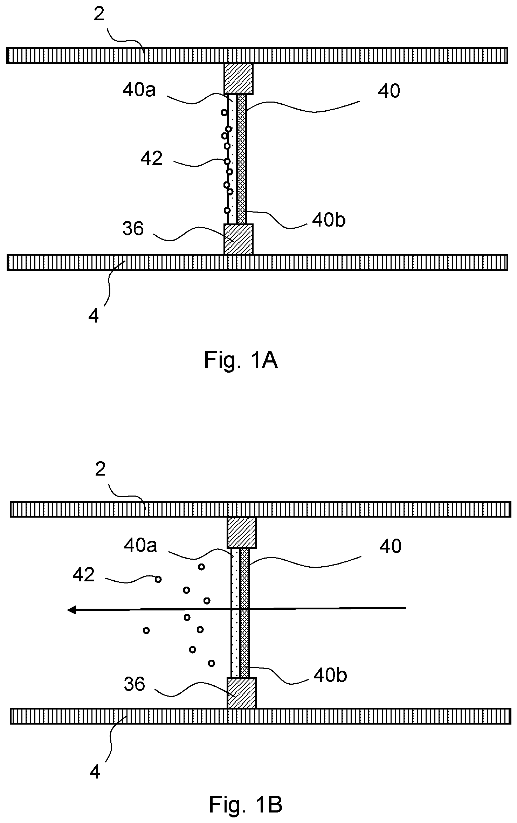

With reference to Figure 1A and 1B the membrane 40 comprises a first portion 40a and a second portion 40b. The first portion 40a comprises the first side 44. The first portion 40a comprises a mesh with an open structure. The second portion 40b comprises a mesh with a closed structure. Accordingly, the mesh of the first portion 40a comprises holes that are larger than the diameter of the particles of active agent and the mesh of the second portion 40b comprises holes that are significantly smaller than the diameter of the particles of active agent. Therefore, the particles of active agent may penetrate into the mesh of the first portion 40a of the membrane 40 and to not penetrate into the mesh of the second portion 40b such that the particles of active agent may not pass through the second portion 40b of the membrane 40.

Prior to use the device 1 is in a first configuration 56 where the first flexible substrate 2 and the second flexible substrate 4 are substantially flat and the support 36 is folded between the first flexible substrate 2 and the second flexible substrate 4.

During use, a user applies force to the first and second edges 6, 8 where the first flexible substrate 2 is connected to the second flexible substrate 4. This application of force flexes the first flexible substrate 2 and the second flexible substrate 4 outward from one another. As the first flexible substrate 2 and the second flexible substrate 4 flex they bend about the first scored line 14 and the second scored line 16 until the device 1 is in the second configuration. Accordingly, a channel 58 is formed between the first flexible substrate 2 and the second flexible substrate 4 having a rectangularly-shaped first end 60 and a biconvex-shaped second end 62 such that the height of the second end 62 is significantly greater that the height of the first end 60, and the width of the second end 62 is significantly less than the width of the first end 60.

In addition, the support 36 unfolds such that the support 36 spans the channel 58 and is located closer to the first end 60 of the channel 58 than to the second end 62 of the channel 58.

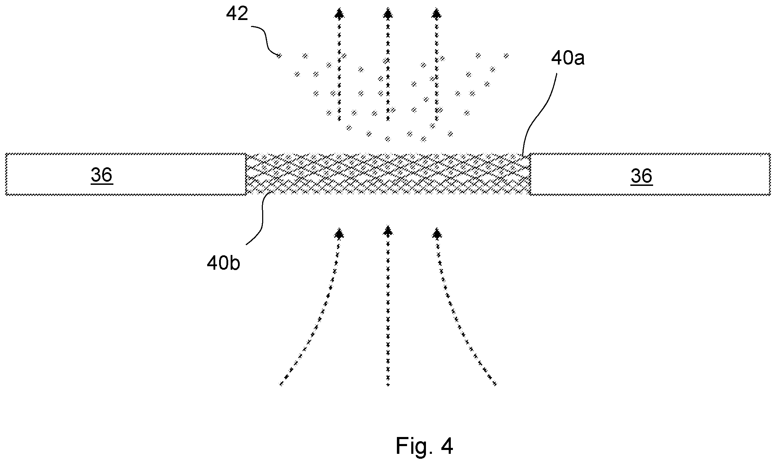

The user then inserts the first end 60 of the device 1 into their mouth and inhales through the device 1. At least a portion of the active agent 42 supported on the membrane 40 spanning the channel 58 is lifted from the membrane 40 and is inhaled by the user. The air flow through the membrane is shown in Figure 4.

Once the active agent 42 has been inhaled the device 1 may be disposed of by the user.

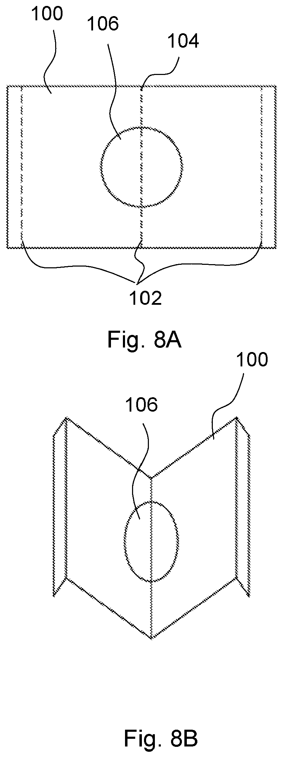

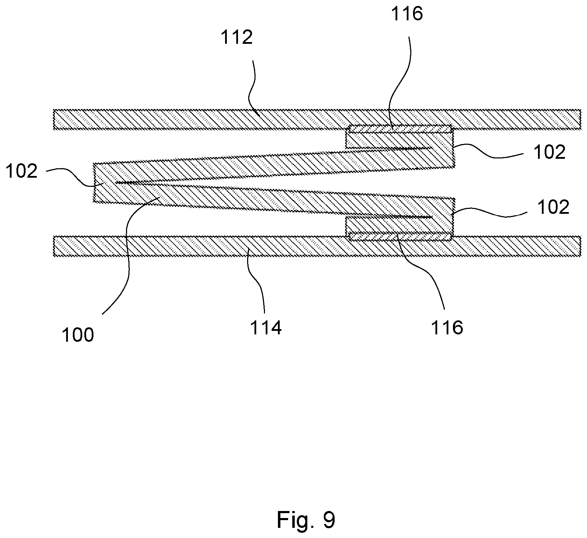

With reference to Figures 8A, 8B and 5, in an alternative embodiment the support 100 comprises three folds 102 with a fold running through the middle 104 of the membrane 106 as shown in Figure 8A and 8B. Figure 9 shows a side view of the support 100 in a folded configuration attached to the first flexible substrate 112 and the second flexible substrate 114 by welds 116.

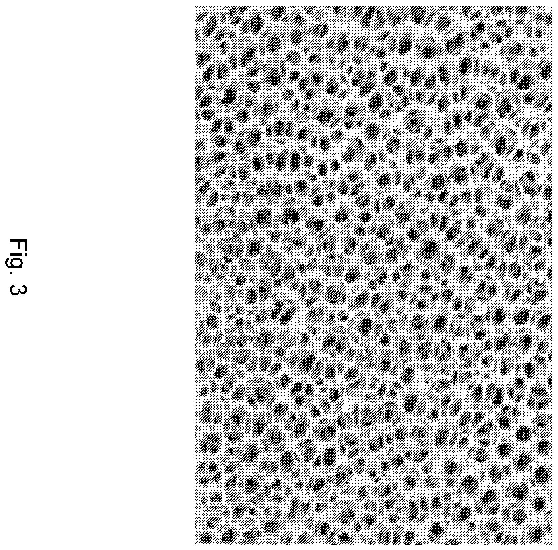

In an alternative embodiment the first portion of the membrane comprises an open cell reticulated foam such as that shown in Figure 3.

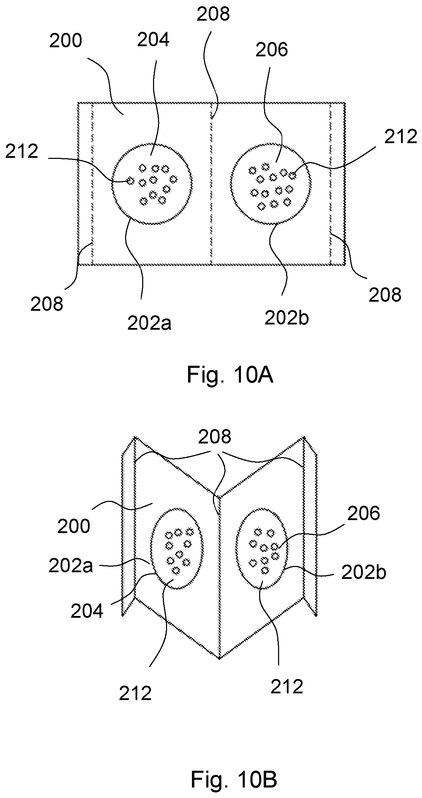

In a further alternative embodiment with reference to Figure 10A and 10B the support 200 comprises a first aperture 202a and a second aperture 202b with a first membrane 204 spanning the first aperture 202a and a second membrane 206 spanning the second aperture 202b. The support 200 comprises three folds 208 with a fold located between the first 202a and second 202b apertures and first 204 and second 206 membranes. The first membrane 204 supports a first active agent 210 and the second membrane 206 also supports the first active agent 210. Accordingly, the device is configured to deliver a higher dose of the first active agent 210 than devices having supports comprising a single membrane.

Alternatively, the first membrane 204 supports a first active agent 212 and the second membrane 206 supports a second active agent 214. Accordingly, the device is configured to deliver two active agents at the same time.

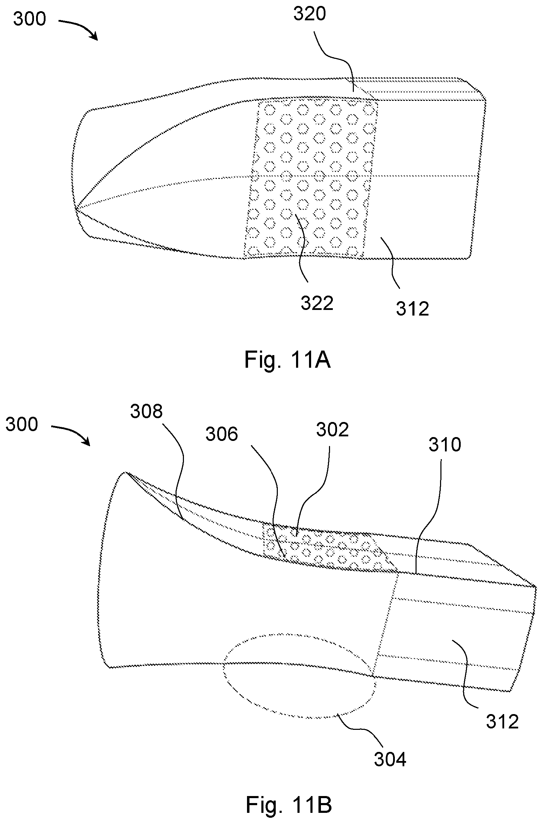

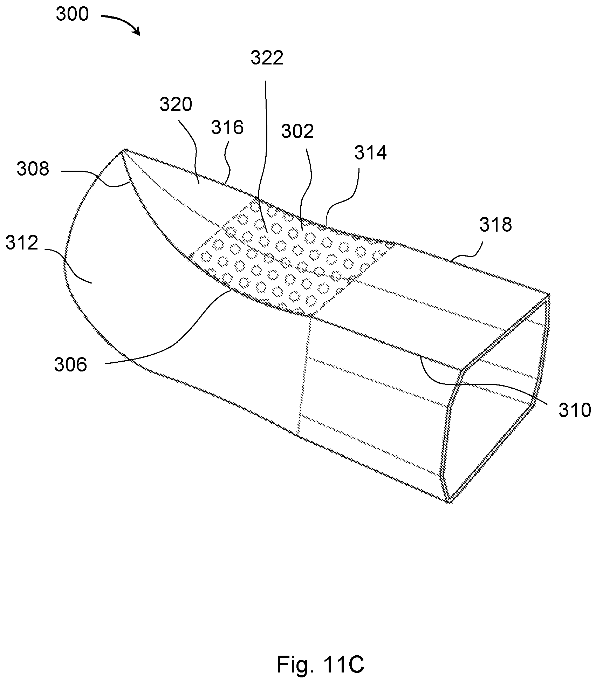

In a still further embodiment with reference to Figures 11A, 11B, and 11C, the device 300 comprises a first locking formation 302 and a second locking formation 304. The first locking formation 302 is formed by a first part 306 of the curved portion 308 of the first scored line 310 of the first flexible substrate 312 and a first part 314 of the curved portion 316 of the first scored line 318 of the second flexible substrate 320. As the device 300 is moved from the first configuration to the second configuration the first locking formation 302 and the second locking formation 304 are pushed by the user until they pop into a locking position. Accordingly, the device is locked into the second configuration such that the user is not required to maintain a force to retain the device 300 in the second configuration.

The first flexible substrate 312 and the second flexible substrate 320 comprises a gripping portion 322. The gripping portion 322 has a textured surface to enhance the ability of the user to grip the device in the second configuration and may act as an indication to the user where force should be applied to move the device from the first configuration to the second configuration.

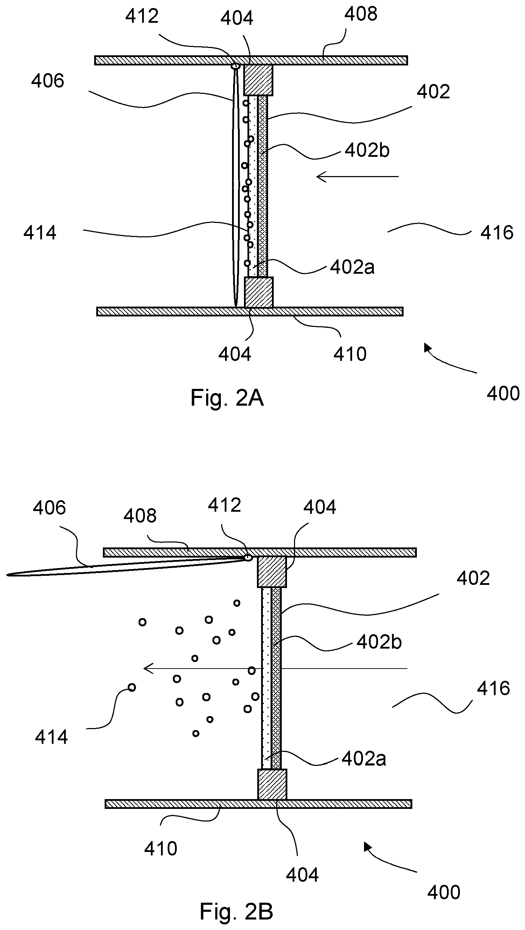

In an alternative embodiment with reference to Figures 2A and 2B, a device 400 according to any of the previously described devices further comprises a membrane 402 comprising a first portion 402a and a second portion 402b mounted within a support 404 and a barrier film 406 (acting as a barrier material) located between a first flexible substrate 408 and a second flexible substrate 410. The barrier film 406 is welded to the first flexible substrate 408 at weld 412 and is releasable attached to the second flexible substrate 410 in the first configuration. An active agent 414 is retained between the barrier film 406 and the membrane 402 prior to use. When the device 400 is moved to the second configuration the membrane 402 occludes the channel 416 formed between the first flexible substrate 408 and the second flexible substrate 410 and the barrier film 406 detaches from the second flexible substrate 410. During use the user inhales through the channel 416 and the barrier film 406 pivots about the weld 412 away from second flexible substrate 410 towards the first flexible substrate 408 to thereby release the active agent 414 from the membrane 402 to the user.

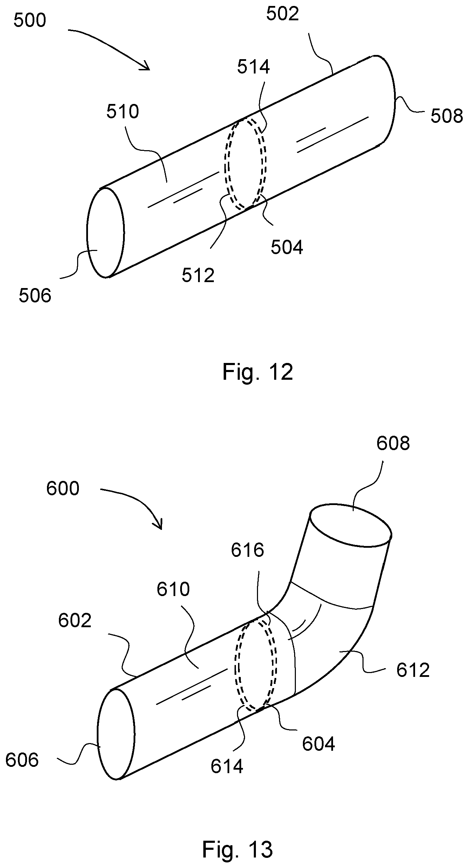

With reference to Figure 12, a device 500 comprises a body 502 and a membrane 504. The body 502 comprises a first opening 506, a second opening 508 and a channel 510 running from the first opening 506 to the second opening 508. The membrane 504 spans the channel 510 and occludes the channel 510. The membrane 504 comprises a first portion 512 facing the first opening 506 and a second portion 514 facing the second opening 508. A particulate active agent is provided in powder form on the first portion 512 of the membrane 504.

The first portion 512 comprises a mesh with an open structure. The second portion 514 comprises a mesh with a closed structure. Accordingly, the mesh of the first portion 512 comprises holes that are larger than the diameter of the particles of active agent and the mesh of the second portion 514 comprises holes that are significantly smaller than the diameter of the particles of active agent. Therefore, the particles of active agent may penetrate into the mesh of the first portion 512 of the membrane 504 and to not penetrate into the mesh of the second portion 514 such that the particles of active agent may not pass through the second portion 514 of the membrane 504.

The first opening 506 and the second opening 508 are covered by a film (not shown) to seal the channel 510 and the membrane 504 spanning the channel 510 from the exterior atmosphere.

Prior to use, the films are removed from the first opening 506 and the second opening 508.

During use the user inhales through the first opening 506 such that the active agent is lifted from the first portion 512 of the membrane 504 and is delivered to the lungs of the user.

With reference to Figure 13, a device 600 comprises a body 602 and a membrane 604. The body 602 comprises a first opening 606, a second opening 608 and a channel 610 running from the first opening 606 to the second opening 608. The body further comprises a flexible portion 612. The membrane 604 spans the channel 610 and occludes the channel 610. The membrane 604 comprises a first portion 614 facing the first opening 606 and a second portion 616 facing the second opening 608. A particulate active agent is provided in powder form on the first portion 614 of the membrane 604.

The first portion 614 comprises a mesh with an open structure. The second portion 616 comprises a mesh with a closed structure. Accordingly, the mesh of the first portion 614 comprises holes that are larger than the diameter of the particles of active agent and the mesh of the second portion 616 comprises holes that are significantly smaller than the diameter of the particles of active agent. Therefore, the particles of active agent may penetrate into the mesh of the first portion 614 of the membrane 604 and to not penetrate into the mesh of the second portion 616 such that the particles of active agent may not pass through the second portion 616 of the membrane 604.

The first opening 606 and the second opening 608 are covered by a film (not shown) to seal the channel 610 and the membrane 604 spanning the channel 510 from the exterior atmosphere.

Prior to use, the films are removed from the first opening 606 and the second opening 608. The body 602 may be bent in the flexible portion 612 such that the first opening 606 may be re-oriented relative to the second opening 608.

During use the user inhales through the first opening 606 such that the active agent is lifted from the first portion 614 of the membrane 604 and is delivered to the lungs of the user.

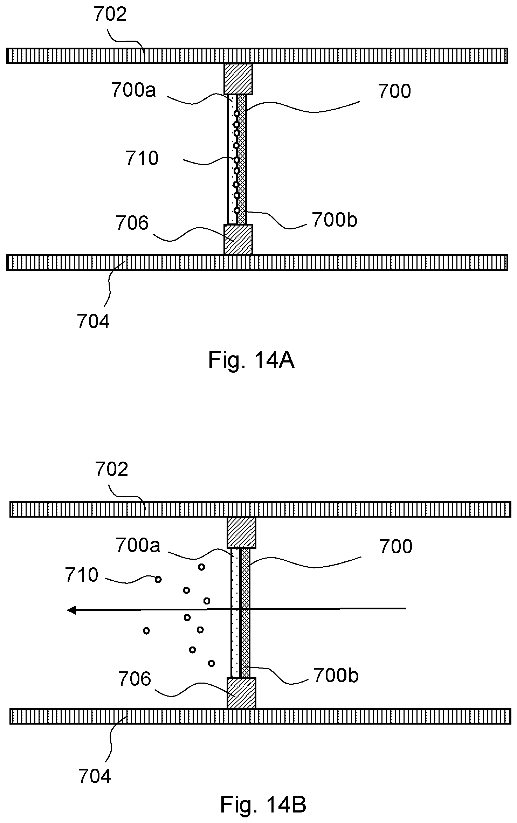

In a further alternative embodiment with reference to Figure 14A and 14B a membrane 700 is provided in a support 706 within a channel defined by channel walls 702, 704 such that the membrane spans the channel. The membrane 700 comprises a first portion 700a and a second portion 700b. The first portion 700a comprises the first side of the membrane. The first portion 700a comprises a mesh with an open structure. The second portion 700b comprises a mesh with a closed structure. Particles of active agent 710 are provided between the first portion 700a and the second portion 700b. Accordingly, the mesh of the first portion 700a comprises holes that are larger than the diameter of the particles of active agent 710 and the mesh of the second portion 700b comprises holes that are significantly smaller than the diameter of the particles of active agent 710. Therefore, the particles of active agent 710 may penetrate into the mesh of the first portion 700a of the membrane 700 and to not penetrate into the mesh of the second portion 700b such that the particles of active agent 710 may not pass through the second portion 700b of the membrane 700.

During use a user inhales through the channel (see Fig. 14B) and the particles of active agent 710 pass through the first portion 700a to the lungs of the user. The first portion 700a prevents passage of agglomerations or clumps of particles of active agent and so acts as a filter to control the particles size of active agent that is delivered to the lungs of the user.

It will be appreciated by the person skilled in the art that the above embodiments are examples and that the features of each disclosed embodiment may be combined with the features of other embodiments.

## Claims

1. An inhaler device (1) comprising two flexible substrates (2, 4) and a membrane (40) located between the two flexible substrates (2, 4), each of the two flexible substrates (2, 4) comprising at least one deformable element (14, 16), the two flexible substrates (2, 4) being connected at two opposing edges (6, 8) and unconnected at two further opposing edges (10, 12), wherein the inhaler device (1) is configured to move between a first configuration (56) where the two flexible substrates (2, 4) are substantially flat and in contact with one another, and a second configuration where the two flexible substrates (2, 4) are flexed such that a channel (58) is formed between the two flexible substrates (2, 4) having a first opening at one end of the channel (58) and a second opening at an opposed end of the channel (58), wherein the membrane (40) is configured to span the channel (58) between the two flexible substrates (2, 4) when the inhaler device (1) is in the second configuration, such that an active agent provided on the membrane (40) may be inhaled by a user when the inhaler device (1) is in the second configuration, wherein the membrane (40) comprises a first portion (40a) facing the first opening and a second portion (40b) facing the second opening, the first portion (40a) being configured to releasably retain the active agent and the second portion (40b) being configured to prevent the passage of the active agent through the second portion (40b),
**characterised in that** the first portion of the membrane comprises a first plurality of apertures or pores, the second portion of the membrane comprises a second plurality of apertures or pores, and the apertures or pores of the first plurality of apertures or pores are larger than the apertures or pores of the second plurality of apertures or pores; or
wherein the first portion (40a) of the membrane (40) and the second portion (40b) of the membrane (40) comprise a mesh, and the mesh of the first portion (40a) of the membrane (40) has larger gaps, holes or voids than the mesh of the second portion (40b) of the membrane (40).

2. The inhaler device (1) of claim 1, wherein the membrane (40) comprises a first major surface and an opposed second major surface and the first portion (40a) of the membrane (40) comprises the first major surface and the second portion (40b) of the membrane (40) comprises the second major surface.

3. The inhaler device (1) of any preceding claim when the first portion of the membrane comprises a plurality of apertures and the second portion of the membrane comprises a second plurality of apertures, wherein the first portion of the membrane is substantially continuous.

4. The inhaler device (1) of any preceding claim when the first portion of the membrane comprises a plurality of apertures and the second portion of the membrane comprises a second plurality of apertures, wherein the second portion of the membrane is substantially continuous.

5. The inhaler device (1) of any preceding claim when the first portion of the membrane comprises a plurality of apertures and the second portion of the membrane comprises a second plurality of apertures, wherein the first portion of the membrane and/or the second portion of the membrane comprises a monolithic material.

6. The inhaler device (1) of any preceding claim, wherein the active agent comprises particles of active agent and the second portion (40b) of the membrane (40) is substantially impermeable to the particles of active agent.

7. The inhaler device (1) of any preceding claim, wherein the first portion (40a) of the membrane (40) comprises the same material as the second portion (40b) of the membrane (40).

8. The inhaler device (1) of any preceding claim, wherein the first portion (40a) of the membrane (40) is less dense than the second portion (40b) of the membrane (40).

9. The inhaler device (1) of any preceding claim, wherein membrane (40) is a laminated membrane comprising two or more layers laminated together.

10. The inhaler device (1) of claim 9, wherein the first portion (40a) of the membrane (40) corresponds to one or more layers of the laminated membrane.

11. The inhaler device (1) of claim 9 or claim 10, wherein the second portion (40b) of the membrane (40) corresponds to one or more layers of the laminated membrane.

12. An inhaler device (500, 600) comprising a membrane (504, 604) located within a channel (510, 610) having a first opening (506, 606) and an opposed second opening (508, 608), wherein the membrane (504, 604) is configured to span the channel (510, 610) such that an active agent provided on the membrane (504, 604) may be inhaled by a user through the channel (510, 610), wherein membrane (504, 604) comprises a first portion (512, 614) facing the first opening (506, 606) and a second portion (514, 616) facing the second opening (508, 608), the first portion (512, 614) being configured to releasably retain the active agent and the second portion (514, 616) being configured to prevent the passage of the active agent through the second portion (514, 616),
**characterised in that** the first portion (512, 614) of the membrane (504, 604) and the second portion (514, 616) of the membrane (504, 604) comprise a mesh, and the mesh of the first portion (512, 614) of the membrane (504, 604) has larger gaps, holes or voids than the mesh of the second portion (514, 616) of the membrane (504, 604).

## Patentansprüche

1. Inhalatorvorrichtung (1), umfassend zwei flexible Substrate (2, 4) und eine Membran (40), die sich zwischen den zwei flexiblen Substraten (2, 4) befindet, wobei jedes der zwei flexiblen Substrate (2, 4) mindestens ein verformbares Element (14, 16) umfasst, wobei die zwei flexiblen Substrate (2, 4) an zwei gegenüberliegenden Kanten (6, 8) verbunden und an zwei weiteren gegenüberliegenden Kanten (10, 12) nicht verbunden sind, wobei die Inhalatorvorrichtung (1) konfiguriert ist zur Bewegung zwischen einer ersten Konfiguration (56), in der die zwei flexiblen Substrate (2, 4) im Wesentlichen flach und in Kontakt miteinander sind, und einer zweiten Konfiguration, in der die zwei flexiblen Substrate (2, 4) gebogen sind, sodass ein Kanal (58) zwischen den zwei flexiblen Substraten (2, 4) gebildet wird, der eine erste Öffnung an einem Ende des Kanals (58) und eine zweite Öffnung an einem gegenüberliegenden Ende des Kanals (58) aufweist, wobei die Membran (40) konfiguriert ist zum Überspannen des Kanals (58) zwischen den zwei flexiblen Substraten (2, 4), wenn sich die Inhalatorvorrichtung (1) in der zweiten Konfiguration befindet, sodass ein Wirkstoff, der auf der Membran (40) bereitgestellt wird, von einem Benutzer inhaliert werden kann, wenn sich die Inhalatorvorrichtung (1) in der zweiten Konfiguration befindet, wobei die Membran (40) einen ersten Abschnitt (40a), der der ersten Öffnung zugewandt ist, und einen zweiten Abschnitt (40b), der der zweiten Öffnung zugewandt ist, umfasst, wobei der erste Abschnitt (40a) dazu konfiguriert ist, den Wirkstoff freisetzbar zurückzuhalten, und der zweite Abschnitt (40b) dazu konfiguriert ist, den Durchgang des Wirkstoffs durch den zweiten Abschnitt (40b) zu verhindern,
**dadurch gekennzeichnet, dass** der erste Abschnitt der Membran eine erste Vielzahl von Aperturen oder Poren umfasst, der zweite Abschnitt der Membran eine zweite Vielzahl von Aperturen oder Poren umfasst und die Aperturen oder Poren der ersten Vielzahl von Aperturen oder Poren größer als die Aperturen oder Poren der zweiten Vielzahl von Aperturen oder Poren sind; oder
wobei der erste Abschnitt (40a) der Membran (40) und der zweite Abschnitt (40b) der Membran (40) ein Netz umfassen und das Netz des ersten Abschnitts (40a) der Membran (40) größere Lücken, Löcher oder Hohlräume als das Netz des zweiten Abschnitts (40b) der Membran (40) aufweist.

2. Inhalatorvorrichtung (1) nach Anspruch 1, wobei die Membran (40) eine erste Hauptoberfläche und eine gegenüberliegende zweite Hauptoberfläche umfasst und der erste Abschnitt (40a) der Membran (40) die erste Hauptoberfläche umfasst und der zweite Abschnitt (40b) der Membran (40) die zweite Hauptoberfläche umfasst.

3. Inhalatorvorrichtung (1) nach einem vorhergehenden Anspruch, wenn der erste Abschnitt der Membran eine Vielzahl von Aperturen umfasst und der zweite Abschnitt der Membran eine zweite Vielzahl von Aperturen umfasst, wobei der erste Abschnitt der Membran im Wesentlichen kontinuierlich ist.

4. Inhalatorvorrichtung (1) nach einem vorhergehenden Anspruch, wenn der erste Abschnitt der Membran eine Vielzahl von Aperturen umfasst und der zweite Abschnitt der Membran eine zweite Vielzahl von Aperturen umfasst, wobei der zweite Abschnitt der Membran im Wesentlichen kontinuierlich ist.

5. Inhalatorvorrichtung (1) nach einem vorhergehenden Anspruch, wenn der erste Abschnitt der Membran eine Vielzahl von Aperturen umfasst und der zweite Abschnitt der Membran eine zweite Vielzahl von Aperturen umfasst, wobei der erste Abschnitt der Membran und/oder der zweite Abschnitt der Membran ein monolithisches Material umfasst.

6. Inhalatorvorrichtung (1) nach einem vorhergehenden Anspruch, wobei der Wirkstoff Wirkstoffpartikel umfasst und der zweite Abschnitt (40b) der Membran (40) für die Wirkstoffpartikel im Wesentlichen undurchlässig ist.

7. Inhalatorvorrichtung (1) nach einem vorhergehenden Anspruch, wobei der erste Abschnitt (40a) der Membran (40) das gleiche Material wie der zweite Abschnitt (40b) der Membran (40) umfasst.

8. Inhalatorvorrichtung (1) nach einem vorhergehenden Anspruch, wobei der erste Abschnitt (40a) der Membran (40) weniger dicht als der zweite Abschnitt (40b) der Membran (40) ist.

9. Inhalatorvorrichtung (1) nach einem vorhergehenden Anspruch, wobei die Membran (40) eine laminierte Membran ist, die zwei oder mehr miteinander laminierte Schichten umfasst.

10. Inhalatorvorrichtung (1) nach Anspruch 9, wobei der erste Abschnitt (40a) der Membran (40) einer oder mehreren Schichten der laminierten Membran entspricht.

11. Inhalatorvorrichtung (1) nach Anspruch 9 oder Anspruch 10, wobei der zweite Abschnitt (40b) der Membran (40) einer oder mehreren Schichten der laminierten Membran entspricht.

12. Inhalatorvorrichtung (500, 600), umfassend eine Membran (504, 604), die sich innerhalb eines Kanals (510, 610) mit einer ersten Öffnung (506, 606) und einer gegenüberliegenden zweiten Öffnung (508, 608) befindet, wobei die Membran (504, 604) konfiguriert ist zum Überspannen des Kanals (510, 610), sodass ein auf der Membran (504, 604) bereitgestellter Wirkstoff von einem Benutzer durch den Kanal (510, 610) inhaliert werden kann, wobei die Membran (504, 604) einen ersten Abschnitt (512, 614), der der ersten Öffnung (506, 606) zugewandt ist, und einen zweiten Abschnitt (514, 616), die der zweiten Öffnung (508, 608) zugewandt ist, umfasst, wobei der erste Abschnitt (512, 614) dazu konfiguriert ist, den Wirkstoff freisetzbar zurückzuhalten, und der zweite Abschnitt (514, 616) dazu konfiguriert ist, den Durchgang des Wirkstoffs durch den zweiten Abschnitt (514, 616) zu verhindern,
**dadurch gekennzeichnet, dass** der erste Abschnitt (512, 614) der Membran (504, 604) und der zweite Abschnitt (514, 616) der Membran (504, 604) ein Netz umfassen und das Netz des ersten Abschnitts (512, 614) der Membran (504, 604) größere Lücken, Löcher oder Hohlräume als das Netz des zweiten Abschnitts (514, 616) der Membran (504, 604) aufweist.

## Revendications

1. Dispositif inhalateur (1) comprenant deux substrats flexibles (2, 4) et une membrane (40) située entre les deux substrats flexibles (2, 4), chacun des deux substrats flexibles (2, 4) comprenant au moins un élément déformable (14, 16), les deux substrats flexibles (2, 4) étant reliés au niveau de deux bords opposés (6, 8) et non reliés au niveau de deux bords opposés supplémentaires (10, 12), ledit dispositif inhalateur (1) étant conçu pour se déplacer entre une première configuration (56) dans laquelle les deux substrats flexibles (2, 4) sont sensiblement plats et en contact l'un avec l'autre, et une seconde configuration dans laquelle les deux substrats flexibles (2, 4) sont fléchis de sorte qu'un canal (58) est formé entre les deux substrats flexibles (2, 4) possédant une première ouverture au niveau d'une extrémité du canal (58) et une seconde ouverture au niveau d'une extrémité opposée du canal (58), ladite membrane (40) étant conçue pour s'étendre sur le canal (58) entre les deux substrats flexibles (2, 4) lorsque le dispositif inhalateur (1) est dans la seconde configuration, de sorte qu'un agent actif disposé sur la membrane (40) peut être inhalé par un utilisateur lorsque le dispositif inhalateur (1) est dans la seconde configuration, ladite membrane (40) comprenant une première partie (40a) faisant face à la première ouverture et une seconde partie (40b) faisant face à la seconde ouverture, la première partie (40a) étant conçue pour retenir de manière libérable l'agent actif et la seconde partie (40b) étant conçue pour empêcher le passage de l'agent actif à travers la seconde partie (40b),
**caractérisé en ce que** la première partie de la membrane comprend une première pluralité d'ouvertures ou de pores, la seconde partie de la membrane comprend une seconde pluralité d'ouvertures ou de pores, et les ouvertures ou les pores de la première pluralité d'ouvertures ou de pores sont plus grands que les ouvertures ou les pores de la seconde pluralité d'ouvertures ou de pores ; ou
dans lequel la première partie (40a) de la membrane (40) et la seconde partie (40b) de la membrane (40) comprennent un maillage, et le maillage de la première partie (40a) de la membrane (40) présente des espaces, des trous ou des vides plus grands que ceux du maillage de la seconde partie (40b) de la membrane (40).

2. Dispositif inhalateur (1) de la revendication 1, dans lequel la membrane (40) comprend une première surface principale et une seconde surface principale opposée et la première partie (40a) de la membrane (40) comprend la première surface principale et la seconde partie (40b) de la membrane (40) comprend la seconde surface principale.

3. Dispositif inhalateur (1) d'une quelconque revendication précédente, lorsque la première partie de la membrane comprend une pluralité d'ouvertures et la seconde partie de la membrane comprend une seconde pluralité d'ouvertures, ladite première partie de la membrane étant sensiblement continue.

4. Dispositif inhalateur (1) d'une quelconque revendication précédente, lorsque la première partie de la membrane comprend une pluralité d'ouvertures et la seconde partie de la membrane comprend une seconde pluralité d'ouvertures, ladite seconde partie de la membrane étant sensiblement continue.

5. Dispositif inhalateur (1) d'une quelconque revendication précédente, lorsque la première partie de la membrane comprend une pluralité d'ouvertures et la seconde partie de la membrane comprend une seconde pluralité d'ouvertures, ladite première partie de la membrane et/ou la seconde partie de la membrane comprenant un matériau monolithique.

6. Dispositif inhalateur (1) d'une quelconque revendication précédente, dans lequel l'agent actif comprend des particules d'agent actif et la seconde partie (40b) de la membrane (40) est sensiblement imperméable aux particules d'agent actif.

7. Dispositif inhalateur (1) d'une quelconque revendication précédente, dans lequel la première partie (40a) de la membrane (40) comprend le même matériau que la seconde partie (40b) de la membrane (40).

8. Dispositif inhalateur (1) d'une quelconque revendication précédente, dans lequel la première partie (40a) de la membrane (40) est moins dense que la seconde partie (40b) de la membrane (40).

9. Dispositif inhalateur (1) d'une quelconque revendication précédente, dans lequel la membrane (40) est une membrane stratifiée comprenant deux couches stratifiées ensemble ou plus.

10. Dispositif inhalateur (1) de la revendication 9, dans lequel la première partie (40a) de la membrane (40) correspond à une ou plusieurs couches de la membrane stratifiée.

11. Dispositif inhalateur (1) de la revendication 9 ou la revendication 10, dans lequel la seconde partie (40b) de la membrane (40) correspond à une ou plusieurs couches de la membrane stratifiée.

12. Dispositif inhalateur (500, 600) comprenant une membrane (504, 604) située à l'intérieur d'un canal (510, 610) possédant une première ouverture (506, 606) et une seconde ouverture (508, 608) opposée, ladite membrane (504, 604) étant conçue pour s'étendre sur le canal (510, 610) de sorte qu'un agent actif disposé sur la membrane (504, 604) peut être inhalé par un utilisateur à travers le canal (510, 610), ladite membrane (504, 604) comprenant une première partie (512, 614) faisant face à la première ouverture (506, 606) et une seconde partie (514, 616) faisant face à la seconde ouverture (508, 608), la première partie (512, 614) étant conçue pour retenir de manière libérable l'agent actif et la seconde partie (514, 616) étant conçue pour empêcher le passage de l'agent actif à travers la seconde partie (514, 616),
**caractérisé en ce que** la première partie (512, 614) de la membrane (504, 604) et la seconde partie (514, 616) de la membrane (504, 604) comprennent un maillage, et le maillage de la première partie (512, 614) de la membrane (504, 604) présente des espaces, des trous ou des vides plus grands que ceux du maillage de la seconde partie (514, 616) de la membrane (504, 604).
